# EUROPEAN PATENT APPLICATION

(11) **EP 4 803 125 A2**
(43) Date of publication of application: **09.09.2026**
(21) Application number: 26177186.9
(22) Date of filing: 28.04.2021
(51) Int. Cl.: A61M 16/10

(54) **RESPIRATORY OR SURGICAL HUMIDIFIER AND METHOD OF USE**

(30) Priority: 29.04.2020 US 202063017455 P
(62) Document number(s) of the earlier application(s) in accordance with Art. 76 EPC: 21797414.6 / 4 142 840
(71) Applicant: Fisher & Paykel Healthcare Limited, East Tamaki, Auckland 2013 (NZ)
(72) Inventor: LIANG, Wenjie Robin, 2013 Auckland (NZ); YU, Yintao, 2013 Auckland (NZ)
(74) Representative: Treeby, Philip David William

(57) **Abstract**

An improved system and method of controlling the operation of a respiratory or surgical humidifier system that can provide humidified gases of substantially constant humidity in dependence upon only one monitored variable, being signals received from a temperature sensor. A power controller can be configured to control a level of power supplied to a heater plate in dependence upon heater plate temperature and a humidity profile so as to drive an operating point of the humidifier towards the humidity profile. The rate of change of a heater plate temperature setpoint can be variably controlled in dependence upon a heater plate temperature setpoint and heater plate temperature.

## Description

### FIELD OF THE DISCLOSURE

The present disclosure relates to respiratory and/or surgical humidifiers, and respiratory or breathing assistance systems for gases to be supplied to a patient or user via a gas supply tube.

### BACKGROUND

Respiratory apparatuses are used in various environments, such as hospital, medical facilities, residential care, palliative care or home environments. For a range of respiratory applications, it is beneficial to humidify gases being supplied to a patient or user. These applications include where the gases are for breathing by the patient or user and/or where the gas is being supplied during surgery to the patient or user. In the case of breathing gases in a non-invasive mode when the inspired gas passes through the upper airway, such as when gas is delivered to the patient or user via a face or nasal mask, the humidity increases patient or user comfort, improves the patient's or user's tolerance to the non-invasive ventilation (NIV), and the humidified gases are less prone to drying out the tissues (for example, the nasal mucosa) of the airway of the patient or user. In the case of surgical gases when the gases are delivered to a surgical site of the patient or an invasive mode when the gases delivered to the patient bypass the upper airway, humidification of the gases has been found to improve patient comfort and provide physiological benefits, such as improved mucus transport, can be necessary for patient or user safety, such as for preventing airway obstruction due to inspissation of airway secretion, disruption of the airway epithelium (or mesothelium in surgical applications), and/or for improving post-operative outcomes. In the case of high flow therapy, humidified gases are delivered to the patient or user at high flows through an unsealed interface. The patient or user may be spontaneously breathing or may be apneic, such as under anesthesia. A flow therapy apparatus with a humidifier can be used to deliver high flow gases and the therapy apparatus may control characteristics such as for example gases flow, including flow rate, temperature, pressure, humidity, supplementary gases concentration, and the like. In the case of positive airway pressure therapy (PAP) therapy, a PAP therapy apparatus that includes a blower and a humidifier can be used to provide pressure therapy, for example, continuous positive airway pressure therapy (CPAP), to the user.

In any of the therapies mentioned above, humidified gases can be delivered to a patient via a gas supply tube. Such gases must be supplied at or near a desired target humidity level. Conventional humidifiers have employed control strategies that are discrete both temporally and in terms of operating points. In such control strategies a number of operating points may be provided defining permitted ranges of heater plate power for an associated heater plate setpoint value. Heater plate temperature is controlled so as to drive the heater plate temperature towards the heater plate setpoint value of the current operating point. The power required by the heater plate is periodically measured and, where this is outside the permitted range for an operating point, operation moves to an adjacent operating point. Many humidifiers also require temperature and/or gas flow sensors within the flow path to perform humidifier control.

### SUMMARY

In a respiratory or surgical humidifier (for humidifying gases for supply to the patient or user), it is important to ensure that the respiratory or surgical humidifier is operating safely and correctly in order to ensure that gases to be supplied to a patient via a gas supply tube are being correctly humidified and that the patient or other user is not exposed to any risk. Accordingly, it is important that humidified gases are maintained at or near a desired target humidity range.

The respiratory or surgical humidifiers and/or methods disclosed herein can have any of the following and/or other advantages. The present disclosure provides examples of respiratory or surgical humidifiers capable of continuously controlling power supplied to a heater plate to deliver a required humidity of humidified gases without requiring monitoring of the flow rate of humidified gases. In some examples the humidifier may continuously control a level of power supplied to the heater plate in dependence upon signals from only one transducer, being signals received from a heater plate temperature sensor, without the need for sensing the flow rate of humidified gases. In some examples the operating point of a humidifier may be continuously controlled towards heater plate temperature and heater plate power supply level combinations of a humidity profile associated with a desired steady state humidity value. In other examples further transducers may be employed such as sensors in the gas flow path, such as sensors that detect the flow rate, pressure, humidity or temperature of the humidified gases or other sensors.

The present disclosure also provides examples of respiratory or surgical humidifiers capable of handling transient events, such as the filling of a humidifier chamber. In some examples the rate of change of a heater plate temperature setpoint may be variably controlled during a transient event.

The examples above can provide relatively simple and inexpensive humidifier systems that are fast to set up and easy to use and require no sensors in the flow path of humidified gases. Enhanced resolution of humidity control may be achieved by continuous control as well as allowing more accurate determination of heater plate temperature setpoint and faster arrival at a desired heater plate temperature setpoint. More consistent humidity levels may be delivered across a range of different breathing circuits and ambient environmental temperatures. Humidified gases having high levels of humidity can be provided whilst reducing condensation and maintaining satisfactory humidifier performance. Improved control over power output to the heater plate can avoid instability during transient events.

The example humidifier systems can provide intrinsically safe operation when the humidifier chamber is empty or when there is no flow of humidified gases through the breathing circuit as control can automatically track down a humidity curve to a low setpoint whereas prior systems may drive to a higher setpoint to try to maintain the temperature of humidified gases at a chamber outlet at a prescribed temperature. Safe operation may also be provided in the case of inlet gases supplied at elevated temperatures, as control is not based on the temperature of inlet gases. The one or more humidity profiles may also be adjusted for ambient temperature providing safe operation for elevated ambient temperatures.

In some configurations, a respiratory or surgical humidifier can comprise a respiratory or surgical humidifier for delivering gases at a desired level of humidity and/or a desired temperature comprising: a housing configured to receive a humidifier chamber; a heating assembly located at least partially within the housing, the heating assembly including: a heat transfer body configured to transfer heat to the humidifier chamber when the humidifier chamber is received by the housing; a heat transfer body temperature sensor configured to sense the temperature of the heat transfer body; and a heater configured to heat the heat transfer body; and a power controller configured to control a level of power supplied to the heater in dependence upon a temperature signal received from the heat transfer body temperature sensor and one or more humidity profiles defining heat transfer body temperature and heater power supply level combinations associated with a desired humidity value.

In some configurations the heat transfer body temperature and heater power supply level combinations of each humidity profile can form a curve associated with a constant humidity value over a desired operating range of the humidifier.

In some configurations each humidity profile can describe a relationship between heat transfer body temperatures and heater power supply levels delivering a desired constant humidity value over a desired operating range of the humidifier.

In some configurations the heat transfer body temperature and heater power supply level combinations of each humidity profile can consist of a plurality of discrete heat transfer body temperature and heater power supply level combinations delivering a desired constant humidity value over a desired operating range of the humidifier.

In some configurations each humidity profile can be selected from one of a number of profiles for different constant humidity values.

In some configurations the respiratory or surgical humidifier can include an ambient temperature sensor.

In some configurations the ambient temperature sensor can be selected from: an infrared detector, a negative temperature coefficient thermistor and a positive temperature coefficient thermistor.

In some configurations each humidity profile can be modified based on ambient temperature.

In some configurations each humidity profile can be scaled based on ambient temperature.

In some configurations the desired humidity value can be a substantially constant steady state humidity value.

In some configurations the respiratory or surgical humidifier can include non-volatile memory storing one or more humidity profiles.

In some configurations the power controller can continuously control the level of power supplied to the heater.

In some configurations the power controller can control the level of power supplied to the heater in dependence upon only one monitored variable, being a temperature signal received from the heat transfer body temperature sensor.

In some configurations the power controller can include a heater control circuit which varies the level of power supplied to the heater at least in part in dependence upon the difference between the temperature signal and a heater plate temperature setpoint value.

In some configurations the heater control circuit can vary the level of power supplied to the heater at least in part in dependence upon proportional and integral components of the difference between the temperature signal and a heater plate temperature setpoint value.

In some configurations the heater control circuit can include a feed forward circuit which modifies the level of power supplied to the heater based on an expected steady state power level for the heater plate temperature setpoint value.

In some configurations the expected steady state power level can be determined by finding the power level associated with the heater plate temperature setpoint value in the humidity profile.

In some configurations the power level from the humidity profile is modified by a derivative value of the heater plate temperature setpoint value.

In some configurations a derivative value of the heater plate temperature setpoint value is added to the power level from the humidity profile.

In some configurations the power controller can include a heater plate temperature setpoint controller that produces a heater plate temperature setpoint value based at least in part upon the heater power supply level and a rate signal based on temperature information.

In some configurations the heater plate temperature setpoint controller can develop a target temperature based on a target temperature associated with the heater power supply level in the humidity profile and modifies the heater plate temperature setpoint based at least in part on the target temperature and the rate signal.

In some configurations the heater plate temperature setpoint controller can determine the difference between a prior heater plate temperature setpoint and a target heater plate temperature setpoint and integrates this difference to produce a new heater plate temperature setpoint value.

In some configurations the heater plate temperature setpoint controller determines the difference between a prior heater plate temperature setpoint and a target heater plate temperature setpoint and combines proportional and integrated components of this difference to produce a new heater plate temperature setpoint value.

In some configurations the heater power supply level supplied to the heater plate temperature setpoint controller is adjusted in dependence upon humidifier chamber fluid level.

In some configurations the heater power supply level supplied to the heater plate temperature setpoint controller is increased with lowering humidifier chamber fluid level.

In some configurations the rate signal modifies the difference between a prior heater plate temperature setpoint and a target heater plate temperature setpoint prior to integration.

In some configurations the rate signal can be based at least in part on the temperature signal and the heater plate temperature setpoint value.

In some configurations the rate signal can reduce the rate of change of the integral component in dependence upon the absolute difference between the temperature signal and heater plate temperature setpoint value.

In some configurations the heater plate temperature setpoint can be a predetermined value at start-up.

In some configurations the heater is a heating element.

In some configurations the heating element is a resistive heating element.

In some configurations the heating element is formed of nichrome wire.

In some configurations the heating element is wound and provided within or in thermal contact with the heat transfer body.

In some configurations the heat transfer body temperature sensor can produce the temperature signal based on a resistance profile of the heating element.

In some configurations the heat transfer body temperature sensor can be a negative temperature coefficient thermistor.

In some configurations the heat transfer body temperature sensor can be a positive temperature coefficient thermistor.

In some configurations the heat transfer body temperature sensor can be a thermocouple.

In some configurations the heat transfer body temperature sensor can be an infrared sensor.

In some configurations the respiratory or surgical humidifier can include two heat transfer body temperature sensors.

In some configurations the power controller can include one or more microprocessor.

In some configurations the heat transfer body can be a heater plate.

In some configurations the respiratory or surgical humidifier can include a humidifier chamber having an inlet for receiving gases and an outlet for supplying humidified gases.

In some configurations a respiratory or surgical humidifier for delivering gases at a desired level of humidity and/or a desired temperature can comprise: a housing configured to receive a humidifier chamber; a heating assembly located at least partially within the housing, the heating assembly including: a heat transfer body configured to transfer heat to the humidifier chamber when the humidifier chamber is received by the housing: a heat transfer body temperature sensor configured to sense the temperature of the heat transfer body and provide a temperature signal; and a heater configured to heat the heat transfer body; and a power controller configured to determine a heater plate temperature setpoint required for the humidifier to deliver gases at a desired level of humidity based at least in part on a level of power supplied to the heater and the temperature signal, wherein a rate of change of the heater plate temperature setpoint is variably controlled in dependence at least in part upon the difference between the heater plate temperature setpoint and the temperature signal; and control the level of power supplied to the heater based on the heater plate temperature setpoint and the temperature signal.

In some configurations the rate of change of the heater plate temperature setpoint can be controlled in dependence upon a rate signal which is based on the current heater plate temperature setpoint and both the temperature signal and an estimated temperature of a fluid within a humidifier chamber when in use.

In some configurations the estimated temperature can be obtained by low pass filtering the temperature signal.

In some configurations the estimated temperature can be obtained by using the heater plate temperature signal when the low pass filtered signal is greater than the heater plate temperature and using the low pass filtered signal when the low pass filtered signal is less than the heater plate temperature.

In some configurations both the temperature signal and the estimated temperature can be compared with the heater plate temperature setpoint to produce error signals which are combined to produce a rate signal controlling the rate of change of the heater plate temperature setpoint.

In some configurations the rate of change of the heater plate temperature setpoint is controlled in dependence upon the current heater plate temperature setpoint and the temperature signal.

In some configurations a polynomial function is applied to the difference between the current heater plate temperature setpoint and the temperature signal and inverted to produce the rate signal.

In some configurations a constant is added to the difference between the current heater plate temperature setpoint and the temperature signal and squared and then inverted to produce the rate signal.

In some configurations the rate signal is filtered.

In some configurations the power controller can include a heater plate temperature setpoint controller that produces a heater plate temperature setpoint value based at least in part upon the heater power supply level and the rate signal.

In some configurations the heater plate temperature setpoint controller can develop a target temperature based on a target temperature associated with a heater power level of the humidity profile and modify the target temperature based at least in part on the rate signal to produce a heater plate temperature setpoint.

In some configurations the heater plate temperature setpoint controller determines the difference between a prior heater plate temperature setpoint and a target heater plate temperature setpoint and integrates this difference to produce a new heater plate temperature setpoint value.

In some configurations the heater plate temperature setpoint controller can determine the difference between a prior heater plate temperature setpoint and a target heater plate temperature setpoint to produce a difference signal and combines proportional and integral components of the difference signal to produce a new heater plate temperature setpoint.

In some configurations rate signal can modify an integral input used to produce the integrated component.

In some configurations the rate signal can reduce the integral input with increasing difference between the heater plate temperature setpoint value and the temperature signal.

In some configurations the rate signal can reduce the integral input with increasing difference between the heater plate temperature setpoint value and both the temperature signal and estimated temperature.

In some configurations the power controller can be configured to control a level of power supplied to the heater in dependence upon temperature signals received from the heat transfer body temperature sensor and one or more humidity profiles defining heat transfer body temperature and heater power supply level combinations associated with a desired humidity value.

In some configurations the heat transfer body temperature and heater power supply level combinations can form a curve associated with a constant humidity value over a desired operating range of the humidifier.

In some configurations the power controller can continuously control the level of power supplied to the heater.

In some configurations the power controller can control the level of power supplied to the heater in dependence upon only one monitored variable, being a temperature signal received from the heat transfer body temperature sensor.

In some configurations a respiratory or surgical humidifier for delivering gases at a desired level of humidity and/or a desired temperature can comprise: a housing configured to receive a humidifier chamber; a heating assembly located at least partially within the housing, the heating assembly including: a heat transfer body configured to transfer heat to the humidifier chamber when the humidifier chamber is received by the housing; a heat transfer body temperature sensor configured to sense the temperature of the heat transfer body and provide a temperature signal; and a heater configured to heat the heat transfer body; and a power controller configured to: determine a heater plate temperature setpoint required for the humidifier to achieve a desired operating point based at least in part on the temperature signal and a level of power supplied to the heater wherein a rate of change of the heater plate temperature setpoint is variably controlled in dependence at least in part upon the difference between the heater plate temperature setpoint and the temperature signal; and control the level of power supplied to the heater in dependence upon the temperature signal and one or more humidity profiles defining heat transfer body temperature and heater power supply level combinations associated with a desired humidity value.

In some configurations a method of controlling a respiratory or surgical humidifier so as to maintain gases at a desired humidity level and/or a desired temperature can comprise controlling the level of power supplied to a heat source heating a liquid in a chamber in dependence upon: power supplied to the heat source; heat source temperature; and one or more humidity profiles defining heat source temperature and heat source power supply level combinations associated with a respective desired humidity value, so as to drive an operating point of the humidifier towards a combination of values of the or a selected humidity profile.

In some configurations the or a selected humidity profile can define a curve and the level of power is controlled to drive an operating point towards the curve.

In some configurations a respiratory or surgical humidifier for delivering gases at a desired level of humidity and/or a desired temperature can comprise: a housing configured to receive a humidifier chamber; and a heater plate assembly located at least partially within the housing, the heater plate assembly including: a heater plate configured to contact a base of the humidifier chamber when the humidifier chamber is received by the housing; a heater plate temperature sensor located at or near the heater plate; and
a heating element configured to heat the heater plate; and a power controller configured to control a level of power supplied to the heating element in dependence upon the temperature signal and one or more humidity profile defining heat transfer body temperature and heater power supply level combinations associated with a respective desired humidity value.

In some configurations a respiratory or surgical humidifier for delivering gases at a desired level of humidity and/or a desired temperature can comprise: a housing configured to receive a humidifier chamber; and a heater plate assembly located at least partially within the housing, the heater plate assembly including: a heater plate configured to contact a base of the humidifier chamber when the humidifier chamber is received by the housing; a heater plate temperature sensor located at or near the heater plate; and a heating element configured to heat the heater plate; and a power controller including: a temperature monitoring circuit configured to determine heater plate temperature based on signals received from the heater plate temperature sensor; memory storing one or more humidity profiles defining heating plate temperature and heating element power supply level combinations associated with a respective desired humidity value; and a power level controller configured to control the level of power supplied to the heating element based upon power level and heater plate temperature values so as to drive an operating point of the humidifier towards the or a selected humidity profile to achieve the desired humidity value.

In some configurations a respiratory or surgical humidifier for delivering gases at a desired level of humidity and/or a desired temperature can comprise: a housing configured to receive a humidifier chamber; and a heater plate assembly located at least partially within the housing, the heater plate assembly including: a heater plate configured to contact a base of the humidifier chamber when the humidifier chamber is received by the housing; a heater plate temperature sensor configured to sense the temperature of the heater plate and provide a temperature signal; and a heating element configured to heat the heater plate; and a power controller configured to: determine a heater plate temperature setpoint required for the humidifier to achieve a desired operating point based on the temperature signals and a power level applied to the heating element by: determining a target temperature of the heater plate based on the power level; determining an error signal based on a difference between the target temperature and the current heater plate temperature setpoint; developing a new heater plate temperature setpoint based on proportional and/or integral values of the error signal, wherein the error signal is modified in dependence upon the heater plate temperature setpoint and the temperature signal before integration; and controlling the level of power supplied to the heating plate based on the heater plate temperature setpoint and temperature signal.

In some configurations a respiratory or surgical humidifier for delivering gases at a desired level of humidity and/or a desired temperature can comprise: a housing configured to receive a humidifier chamber; and a heater plate assembly located at least partially within the housing, the heater plate assembly including: a heater plate configured to contact a base of the humidifier chamber when the humidifier chamber is received by the housing; a heater plate temperature sensor configured to sense the temperature of the heater plate and provide a temperature signal; and a heating element configured to heat the heater plate; and a power controller configured to: determine a heater plate temperature setpoint required for the humidifier to achieve a desired operating point based on temperature signals and a power level applied to the heating element by: determining a target temperature of the heater plate based on a defined relationship with the power signal;
determining an error signal based on a difference between the target temperature and the temperature signal; and developing a new heater plate temperature setpoint based on proportional and/or integral values of the error signal, wherein the error signal is modified in dependence upon the heater plate temperature setpoint and the temperature signal before being integrated; and controlling the level of power supplied to the heating element based on the heater plate temperature setpoint and temperature signal.

In some configurations a respiratory or surgical humidifier for delivering gases at a desired level of humidity and/or a desired temperature can comprise: a heater configured to transfer heat to a humidifier chamber; a temperature sensor configured to sense the temperature of the heater; and a power controller configured to continuously control a level of power supplied to the heater in dependence upon only one monitored variable, being signals received from the temperature sensor.

In some configurations the power controller can store one or more humidity profiles defining heater temperature and heater power supply level combinations associated with a desired humidity value and drives operation of the humidifier towards values stored in the humidity profile.

In some configurations the desired level of humidity can be a desired level of absolute humidity

Where the term "profile" is used in this specification it refers to values of two or more attributes satisfying a requirement of a further attribute and may be a continuous or discontinuous representation of values, such as a continuous curve or values stored in a look up table or a relationship producing such values, such as a polynomial equation.

Where the construction "and/or" is used it refers to the inclusive form of "or" known as the Boolean OR operator, meaning "(and) or (or)".

"Steady state" is an equilibrium condition of a circuit that occurs when the effects of transients are no longer apparent.

### BRIEF DESCRIPTION OF THE DRAWINGS

These and other features, aspects, and advantages of the present disclosure are described with reference to the drawings of certain embodiments, which are intended to schematically illustrate certain embodiments and not to limit the disclosure.
Figures 1A and 1B illustrate schematically example surgical humidifier systems.
Figure 1C illustrates schematically an example respiratory humidifier system.
Figure 1D illustrates schematically an example heater base unit of the respiratory humidifier system of Figure 1C.
Figure 1E illustrates schematically an example respiratory humidifier system.
Figure 1F illustrates schematically an example heater base unit of the respiratory humidifier system of Figure 1E.
Figure 1G illustrates schematically a partial view of the heater base unit and an example breathing circuit tube heating element adapter of Figure 1E.
Figure 2 illustrates a series of curves showing the relationship between heater plate temperatures and power supply levels for various gas flow rates.
Figure 3 illustrates a constant humidity curve superimposed upon the curves shown in figure 2.
Figure 4 illustrates a control method based upon a constant humidity curve.
Figure 5 illustrates the variance of constant humidity curves with changing ambient temperature.
Figure 6 illustrates a control diagram of an example power controller.
Figure 7A illustrates a control diagram of an example heater plate power control section of the power controller shown in Figure 6.
Figure 7B illustrates a control diagram of an alternate example heater plate power control section of the power controller shown in Figure 6.
Figure 8A illustrates a control diagram of another example power controller.
Figure 8B illustrates a control diagram of another example power controller.
Figure 9 illustrates a control diagram of an example temperature setpoint control section of the power controller shown in Figure 8 or 8A.
Figure 10 illustrates a control diagram of an example rate control section of the power controller shown in Figure 8 or 8A.
Figure 11 illustrates a control diagram of another example rate control section of the power controller shown in Figure 8 or 8A.
Figure 12 illustrates measured and estimated heater plate temperature values.
Figure 13 illustrates an example control strategy using measured and estimated heater plate temperature values.

### DETAILED DESCRIPTION

Although certain embodiments and examples are described below, those of skill in the art will appreciate that the disclosure extends beyond the specifically disclosed embodiments and/or uses and obvious modifications and equivalents thereof. Thus, it is intended that the scope of the disclosure herein disclosed should not be limited by any particular embodiments described below. For example, component values and operating parameters are examples only and are not limiting.

### Example Respiratory or Surgical Humidifier

The present disclosure provides examples of a respiratory humidifier configured to supply humidified and/or heated gas to a patient or user in multiple modes. The modes for the respiratory humidifier can include at least an invasive mode (for example, for patients with a bypassed airway) and a non-invasive mode (for example, for patients or users with breathing masks). Each mode can have a number of humidity settings, which can be expressed as a dew point or absolute humidity. The respiratory humidifier is controlled to deliver, at an outlet of the humidification chamber and/or the patient end of the gas supply tube, humidified gases having a dew point (or absolute humidity) at or near a predetermined humidity level. For example, a user can select a setting appropriate for the current mode of operation. A number of target humidity settings may be provided, for example, the humidity settings may be equivalent to a dew point of about 37 degrees Celsius, about 31 degrees Celsius, about 29 degrees Celsius, about 27 degrees Celsius, or others and each target humidity setting may have an acceptable tolerance range. The humidity setting equivalent to a dew point of 37 degrees Celsius may be suitable for invasive therapy (i.e., where the patient's upper airways are bypassed) whereas the other humidity settings may be suitable for non-invasive therapy, although the humidity settings may not be restricted to a particular type of therapy. For example, a user can select a humidity setting of about 34 degrees Celsius which may be suitable for both invasive or non-invasive therapy. Alternatively, each humidity setting may be continuously variable between upper and lower limits. A lower humidity setting may be selected by the user to reduce condensation or "rain-out" in the gas supply tube, or a higher humidity setting may be selected to improve patient comfort or physiological benefits. Some respiratory humidifier systems disclosed herein can also include a high flow, unsealed mode or any other modes known to those of skill in the art. High flow therapy as discussed herein is intended to be given its typical ordinary meaning, as understood by a person of skill in the art, which generally refers to a respiratory assistance system delivering a targeted flow of humidified respiratory gases via an intentionally unsealed patient interface with flow rates generally intended to meet or exceed inspiratory flow of a patient. Typical patient interfaces include, but are not limited to, a nasal or tracheal patient interface. Typical flow rates for adults often range from, but are not limited to, about fifteen liters per minute to about sixty liters per minute or greater. Typical flow rates for pediatric patients (such as neonates, infants and children) often range from, but are not limited to, about one liter per minute per kilogram of patient weight to about three liters per minute per kilogram of patient weight or greater. High flow therapy can also optionally include gas mixture compositions including supplemental oxygen and/or administration of therapeutic medicaments. High flow therapy is often referred to as nasal high flow (NHF), humidified high flow nasal cannula (HHFNC), high flow nasal oxygen (HFNO), high flow therapy (HFT), or tracheal high flow (THF), among other common names. For example, in some configurations, for an adult patient 'high flow therapy' may refer to the delivery of gases to a patient at a flow rate of greater than or equal to about 10 litres per minute (10 LPM), such as between about 10 LPM and about 100 LPM, or between about 15 LPM and about 95 LPM, or between about 20 LPM and about 90 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient 'high flow therapy' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow therapy apparatus with an adult patient, a neonatal, infant, or child patient, may deliver gases to the patient at a flow rate of between about 1 LPM and about 100 LPM, or at a flow rate in any of the sub-ranges outlined above.

High flow therapy can be effective in meeting or exceeding the patient's inspiratory demand, increasing oxygenation of the patient and/or reducing the work of breathing. Additionally, high flow therapy may generate a flushing effect in the nasopharynx such that the anatomical dead space of the upper airways is flushed by the high incoming gases flow. The flushing effect can create a reservoir of fresh gas available of each and every breath, while minimizing re-breathing of carbon dioxide, nitrogen, etc.

The patient interface for use in a high flow therapy can be a non-sealing interface to prevent barotrauma, which can include tissue damage to the lungs or other organs of the patient's respiratory system due to difference in pressure relative to the atmosphere. The patient interface can be a nasal cannula with a manifold and nasal prongs, and/or a face mask, and/or a nasal pillows mask, and/or a nasal mask, and/or a tracheostomy interface, or any other suitable type of patient interface.

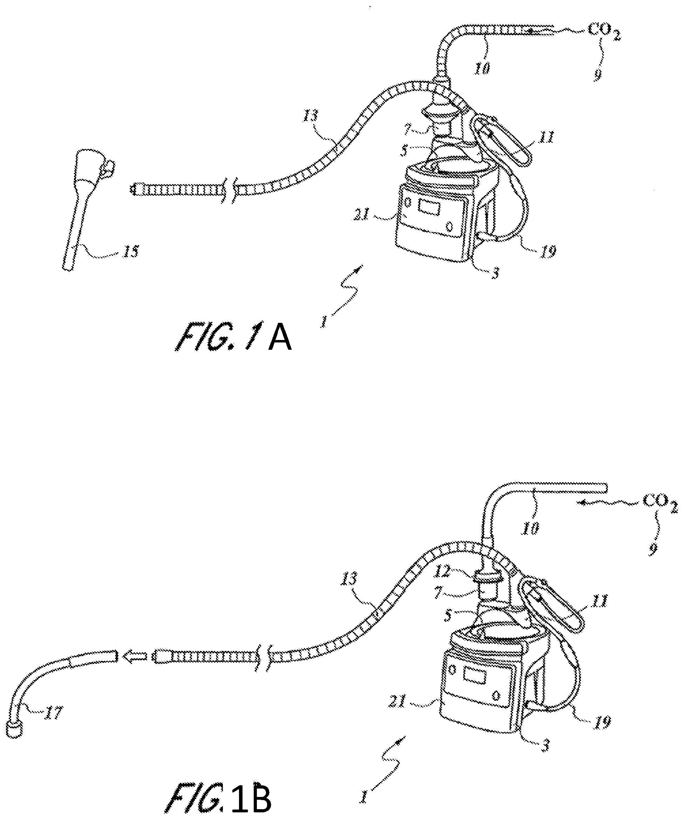

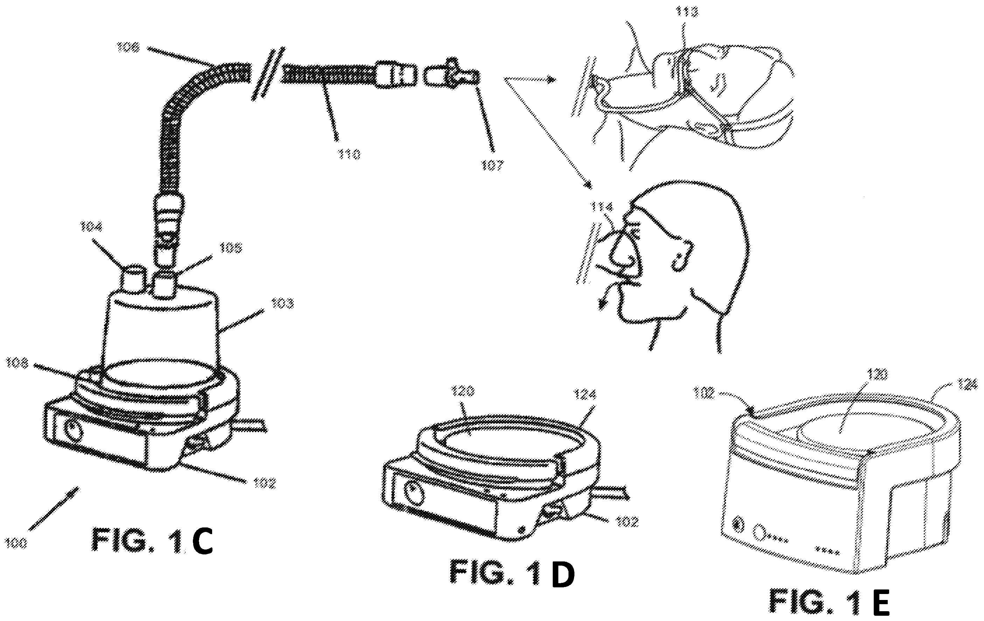

The circuits and methods described below in relation to respiratory humidifier with reference to figures 1C to 13 may be similarly applied in a surgical humidifier which may be used, for example, in laparoscopic surgery. Figures 1A and 1B are schematic views of example embodiments of a surgical humidification and gas delivery apparatus 1. The apparatus 1 comprises a base unit 3 and a humidification chamber 5 removably mounted on the base unit 3. The humidification chamber 5 comprises a gas inlet 7 arranged to be connected to a gas source 9 via an inlet conduit 10 to deliver the gas, for example carbon dioxide, into the chamber 5. The chamber 5 further comprises a gas outlet 11 arranged to be connected to a gas delivery conduit 13 to deliver humidified gas to a patient. A filter 12 (shown in Figure 1B) may be provided between the gas source 9 and the chamber 5 to filter the incoming gas.

In the embodiment of Figure 1A, an end of the gas delivery conduit 13 comprises a trocar 15 arranged to be connected to a patient for use in closed medical procedures such as endoscopy and laparoscopy. In the embodiment of Figure 1B, an end of the gas delivery conduit 13 comprises a diffuser 17 arranged to diffuse the humidified gas into a wound of a patient during open medical procedures, such as open surgery.

The apparatus 1 comprises, for example, a heater. The heater can comprise a heater plate on the base unit 3, for example. The heater is configured to heat humidification liquid in the chamber 5 to generate vapor. The humidification liquid is typically, but not necessarily, water. Gas from the gas source 9 flows into the chamber 5 and passes over the heated humidification liquid, thus taking up vapor and increasing in humidity level prior to delivery to the patient via the gas delivery conduit 13. The chamber 5 may alternatively or additionally comprise an integral heater or a heater located inside the chamber 5.

The gas delivery conduit 13 can also comprise or be provided with a heater. A heater for the gas delivery conduit 13 can ensure that the gas temperature is maintained at a desired level along the conduit 13 as well as minimize or eliminate the formation of condensation. A heater for the gas delivery conduit 13 can have a resistance wire provided in or attached to the conduit 13, or a wire or other heater element provided inside the conduit 13. A heater for the gas delivery conduit 13 may be electronically connected to the base unit 3 or to the chamber 5, for example by an electrical cable 19 to power the heater. Additionally or alternatively, the conduit 13 may be thermally insulated.

The apparatus 1 comprises a controller 21 arranged to control the apparatus 1, and in particular to control the flow rate, temperature, and humidity of gas delivered to the patient to be appropriate for the type of medical procedure for which the apparatus is being used. The controller 21 therefore controls, among other things, a heater for the humidification chamber 5 and/or a heater for the gas delivery conduit 13, if provided. The controller 21 can also control a regulator that regulates the flow rate of gas through the apparatus 1. The regulator may comprise a flow inducer and/or inhibiter such as a motorized fan. Valves and/or vents may additionally or alternatively be used to control flow rate. The controller 21 may comprise an electronic controller, that may be microprocessor-based, for example. The system can comprise memory and any electronic components capable of performing calculations as would be understood by those of skill in the art.

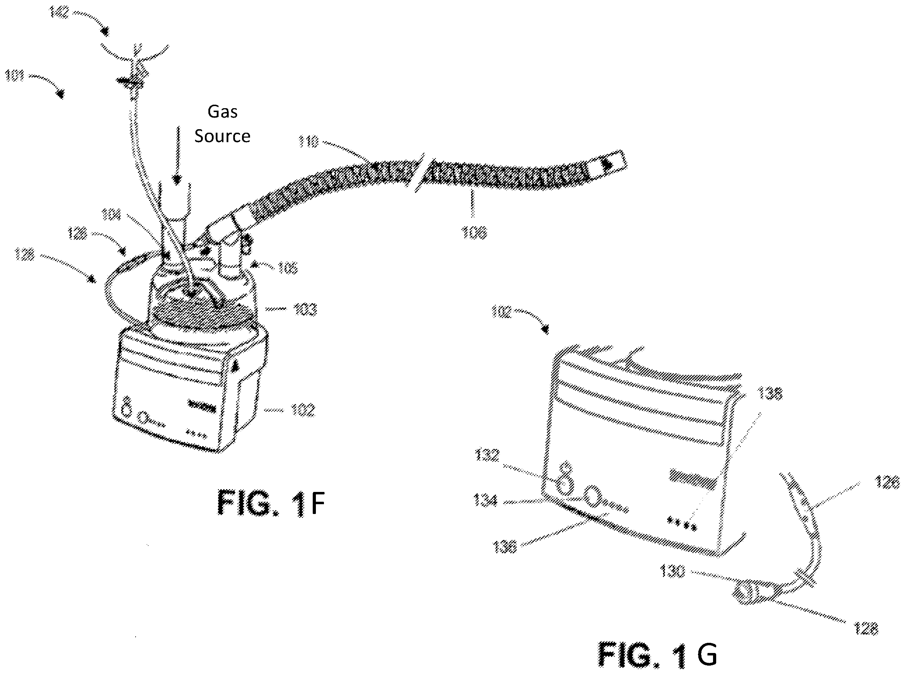

Referring to Figures 1C and 1E, an example respiratory humidifying system 100 can include a heater base unit 102 having a heat transfer body, which in this example is in the form of heater plate 120 (see Figures 1D and 1F) having a substantially planar top surface. The heat transfer body may take different shapes including shapes that at least partially encompass the humidifier chamber 103. The heater plate 120 can have an electric heating element therein or in thermal contact therewith. Optionally one or more electrical insulation layers can be located between in the heater plate and the heater element. The heater element can be a base element (or a former) with a wire wound around the base element. The wire can be a nichrome wire (also known as nickel-chrome or chrome-nickel, being any of various alloys of nickel and chromium and sometimes other elements). The heater element can also include a multi-layer substrate with heating tracks electrodeposited thereon or etched therein. The heater base unit 102 can have a housing and a controller (for example, a microprocessor-based controller) contained within the housing for controlling the supply of power to the heating element(s) of the heater plate 120.

The humidifier heater plate 120 can have a heater plate temperature sensor. An analog or digital temperature sensor may be used. The temperature sensor may be a temperature transducer, thermocouple, infrared sensor, a temperature sensor that produces a temperature signal based on the resistance profile of the heating element, a negative temperature coefficient thermistor, a positive temperature coefficient thermistor or other suitable type of sensor. The temperature sensor can measure a temperature of the heater plate 120. The temperature sensor can be in electrical communication with the controller in the heater base unit 102 so that the controller can monitor the temperature of the heater plate 120.

The humidifier chamber 103 can be removably received and retained on the heater base unit 102, such that the humidifier chamber base is positioned in contact with the heater plate 120 in the heater base unit 102. Referring to Figures 1D and 1F, which illustrate examples of the heater base unit 102 of Figures 1C and 1E respectively, the humidifying base 102 can have a collar 124 for engaging with a flange on the humidifier chamber 103, such as shown in Figures 1C and 1E. The collar 124 defines a lip that engages a flange of the humidifier chamber 103 to retain the humidifier chamber 103 in an operative position on the heater base 102. The humidifier chamber 103 can include a thermally conductive base. When engaged with the heater base unit 102, the conductive base of the humidifier chamber 103 can be in contact with the heater plate 120, such as an upper surface of the heater plate 120. Water inside the chamber 103 is heated when a power signal is sent to the heating element to energize the heating element. The chamber 103 can also be connected to a water source 142 (Figure 1E), which can add water to the chamber 103 when the water is low or completely out in the chamber 103. Adding of water can be manually performed, such as upon a warning from the system 101 that there may be a low water or water-out condition, or automatically performed, such as using a float valve connected to a water supply.

With continued reference to Figures 1C and 1E, the gases to be humidified can include one or more of air, oxygen, anesthetic, other auxiliary gases, or any mixture of gases. The gases can be supplied to the humidifier chamber 103 through a gases inlet 104, which can be connected to a gas source, such as a ventilator, in the case of CPAP therapy a CPAP blower, or a remote source. For high flow therapy, a blower or further alternatively a wall source with a flow and/or pressure regulator can supply the gases. The humidifier chamber 103 also includes a gases outlet 105, which can connect to a breathing circuit 106. The breathing circuit 106 can convey humidified and heated gases to a patient or user. As shown in Figure 1C, a patient end 107 of the breathing circuit 106 can connect to a patient interface, such as a nasal cannula 113 or a nasal mask 114. The breathing circuit 106 can also connect to other types of patient or user interfaces, such as a full-face mask, total-face mask, nasal pillows mask, endotracheal tube, or others. The breathing circuit 106 of Figure 1E can similarly be connected to any suitable patient interface. The breathing circuit 106 may include a gas supply tube with or without a tube heating element. The respiratory humidifying system 100, 101 may be configured for use with both heated and unheated gas supply tubes or breathing circuits.

A tube heating element 110 (such as one or more heater wires embedded in the gas supply tube wall, contained within the gas supply tube, or wrapped around the outside of the gas supply tube) can be provided in the breathing circuit 106. The tube heating element 110 reduces condensation and ensures the temperature and/or humidity of gases is maintained in a predetermined range, for example keeping the temperature of gases in the tube above a selected dew point. The tube heating element 110 can also optionally be in electrical communication with the controller in the heater base unit 102. As shown in Figures 1E and 1G, a breathing circuit tube heating element adaptor cable 128 can have two connectors at two ends of the cable 128 for coupling the tube heating element 110 to the heater base unit 102 (such as to the controller of the heater base unit 102). Alternatively, the tube heating element adapter cable may be permanently electrically connected to a tube heating element supply circuit of the heater base unit 102, for example by soldering. The tube heating element adaptor cable 128 can facilitate an easy connection between the tube heating element 110 and the heater base unit 102. The tube heating element 110 is controlled by the controller, including the controlling of power to the tube heating element 110. The tube heating element adaptor cable 128 can also include an ambient temperature sensor 126 (which may be an infrared detector, a negative temperature coefficient thermistor or a positive temperature coefficient thermistor), which can allow the system 101 to adjust the tube heating element 110 power and/or heater plate power to compensate for ambient temperatures or changes in the ambient temperature. The ambient temperature sensor can alternatively be located anywhere that is exposed to the ambient air. A tube heating element indicator 130 can be embedded into the connector that couples to the heater base unit 102. The tube heating element indicator 130 can be illuminated when a properly functioning tube heating element 110 is connected to the heater base unit 102, and the system 101 can heat the gas inside the breathing circuit 106 via the tube heating element 110 to minimize condensate in addition to heating the gas passing through the humidifier chamber 103 via the heater plate 120. If the tube heating element 110 is malfunctioning or not connected, the tube heating element indicator 130 is not illuminated (or is flashing), and the system 101 may heat the gas only by heating the water in the chamber 103 via the heater plate 120. Alternatively, the tube heating element indicator 130 may be illuminated when there is a fault or a disconnection of the adaptor cable 128 from the tube heating element 110. The illuminated indicator 130 can act as a visual message or a visual warning. The indicator 130 may not be illuminated if the tube heating element 110 is functioning correctly.

The controller of the respiratory humidifier system 100 can control at least the heater plate 120, and preferably or optionally also the tube heating element 110, without additional sensors (for example, in the humidifier chamber, at the chamber outlet, in the breathing circuit, and/or elsewhere in the system). This can be achieved by estimating a heater plate operating point required to deliver a required humidity. For a given respiratory humidifier system, the controller can determine an appropriate level of power to apply to the heater plate 120. Applying power to the heater 120 can generate humidity and heat the gases. The heater plate power and temperature can be controlled to generate a predetermined amount of humidity. Additionally, the parameters can also optionally be used by the controller to provide a more appropriate level of energization to the tube heating element 110. As shown in Figures 1E and 1G, the system 101 can also include the ambient temperature sensor 126. The ambient temperature sensor can be located anywhere that is exposed to the ambient air. For example, the system 101 can include the ambient temperature sensor 126 on the tube heating element adaptor cable 128.

As shown in Figure 1G, a front panel of the heater base unit 102 can include a plurality of user controls and indicators, such as a power button 132, a humidity setting push button 134, and a plurality of (such as three, four, five or more) humidity settings indicators 136 (which can include LED lights) next to the humidity setting push button 134. The locations, shapes, and sizes of the user controls and indicators are not limiting. There can be four levels of humidity settings available which are indicated by the four humidity setting indicators 136. In another example the four, or other selected number of, humidity settings may correspond to a number of different types of therapies provided to a patient in a selected therapy mode. Some or all of the humidity settings may, for example, be selectable when using either an invasive or a non-invasive therapy mode. For example, the highest amount of humidity can be selected when the humidifier is operating in an invasive therapy mode. The lowest amount of humidity may be applied in a non-invasive therapy mode. The amount of humidity can be selected based on therapeutic requirements or therapy type, or it may be predefined. Alternatively, the heater base unit 102 may include a controller that is configured to automatically select the amount of humidity to be delivered based on a therapy mode, the patient, or the type of therapy being applied to the patient. Optionally, the heater base unit 102 may include a display or touch screen that may communicate information to the user. The touch screen may also be configured to receive inputs from the user.

The humidity level can be adjusted by pressing the humidity settings push button 134, which can also be a momentary push button. The front panel can also include a plurality of alarm indicators 138 (which can include LED lights) to indicate the following nonlimiting examples of conditions: "water out" condition (including low water and water-out), tube heating element adaptor not connected, audible alarm muted, and a "See Manual" indication used to indicate that a fault has occurred within the system 101.

The system 101 can be suitable for providing respiratory therapy for different purposes, such as for critical care (for example, in the hospital) and home care. The system 101 is suitable for providing invasive, non-invasive and high flow therapies for both adult and pediatric patients.

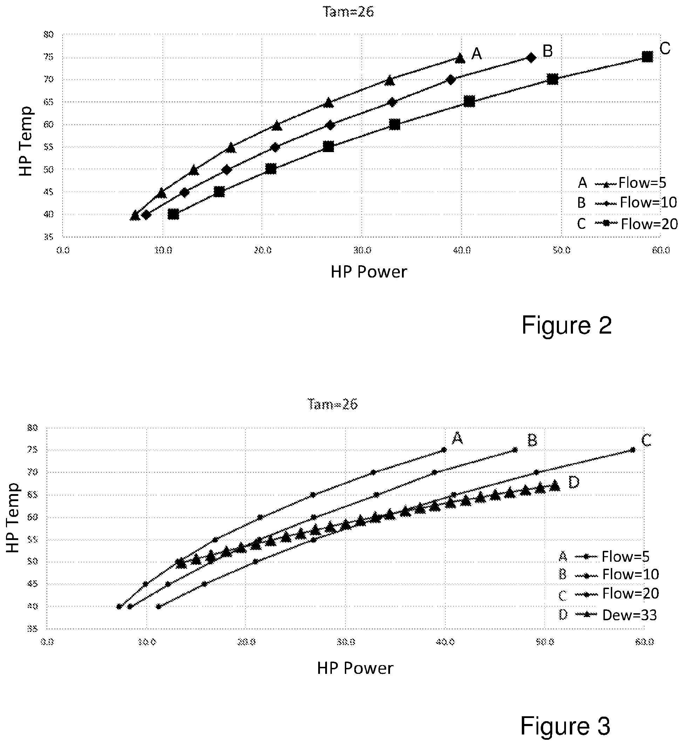

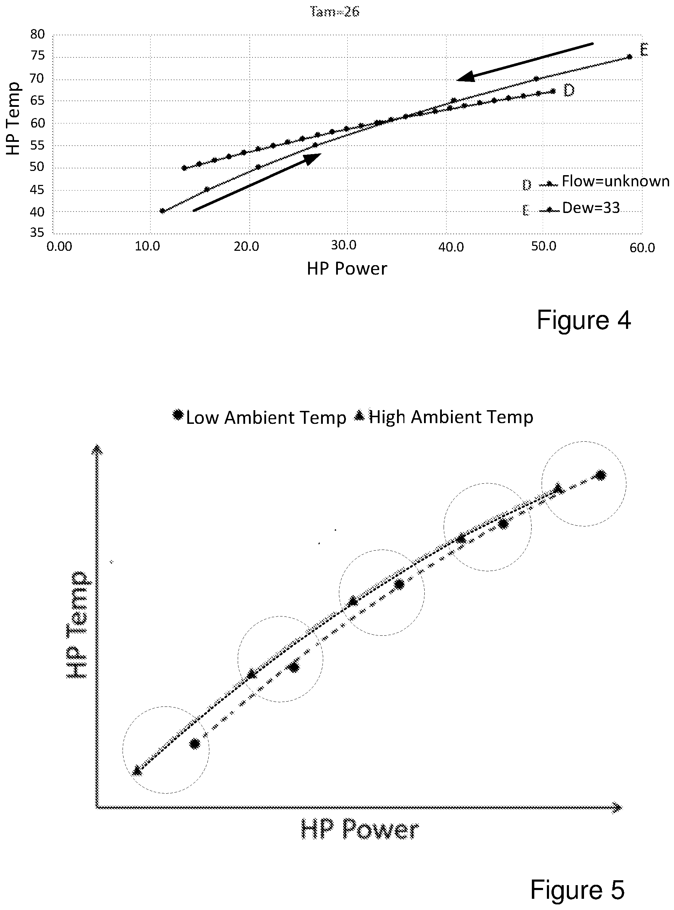

Figure 2 shows a series of curves A, B and C showing the relationship between heater plate temperature and the power supplied to a heater plate for three different flow rates producing different humidity levels at different points along each curve. Along each curve A, B and C is a single operating point that will deliver a desired humidity level in steady state operation. With reference to Figures 3 to 5, a novel heater plate power control method will be described. Curve D (see Figure 3) passes through the single operating point of each curve A, B and C producing humidified gases having a desired constant absolute humidity and extends this to all flow rates within a desired operational range of the humidifier. It has now been appreciated that curve D defines steady state heater plate temperatures and power levels producing humidified gases of a desired constant humidity. With this insight it has been realized that it is not necessary to determine gas flow rate to determine a target operating point and that continuous control may be performed based on the constant humidity curve D (referred to in the following as a "humidity profile") for a selected absolute humidity value. As illustrated in Figure 4, any arbitrary flow rate E will intersect constant humidity curve D and by driving operation of the humidifier towards curve D (i.e., by increasing or decreasing the heater plate power as indicated by the arrows) will bring operation to a steady state power level and heater plate temperature combination required to deliver a desired target humidity level. In operation if heater plate power and heater plate temperature combinations depart from curve D a target temperature for the heater plate (heater plate setpoint) may be adjusted to bring the operating point back towards curve D. Assuming a humidifier is operating initially at A, a humidity profile may be produced using heater plate temperature, or a value indicative of heater plate temperature or derivable from heater plate temperature, and power supply levels to a heater plate that form a curve associated with a constant humidity value over a desired operating range of a humidifier.

As illustrated in a further refined embodiment shown in Figure 5 it may be seen that humidity curves have a slight dependence upon ambient temperature. Pairs of points along a low ambient temperature curve (solid circles) and points along a high ambient temperature curve (triangles) having the same flow and humidity output are indicated within dashed circles. For a given set of conditions, a higher ambient temperature results in less heat being dissipated from the system to the ambient environment and therefore a lower operating point is required to output the same humidity. Conversely, a lower ambient temperature results in more heat being dissipated from the system to the ambient environment and therefore requires a higher operating point to output the same humidity. It will be seen that the high ambient temperature value of each pair shifts down and left with increasing ambient temperature. This variance may be compensated for by scaling humidity curve D in dependence upon ambient temperature, or using another compensation technique.

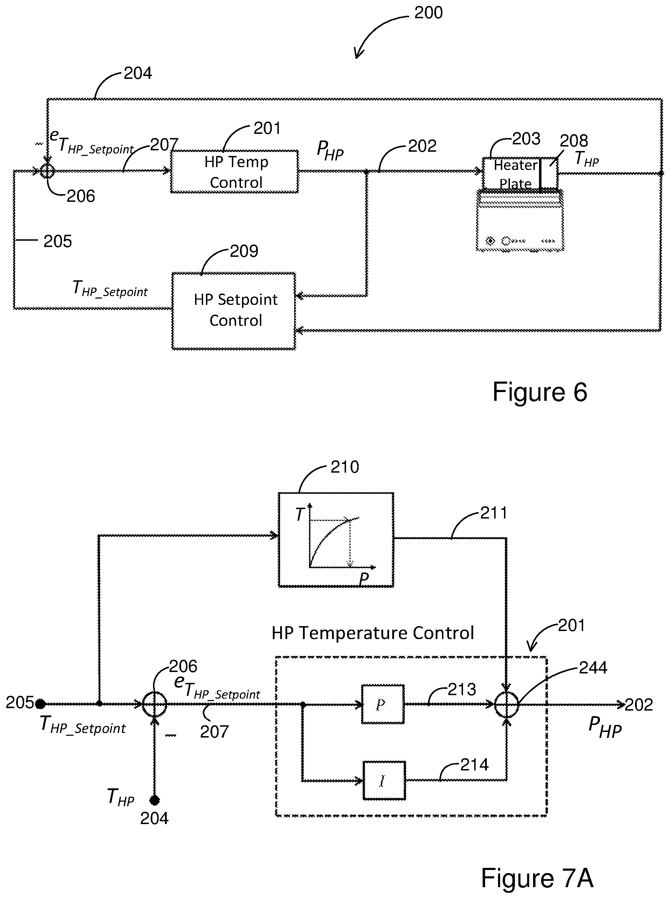

With reference to Figure 6, an example control diagram for a humidity controller 200 for a respiratory or surgical humidifier is shown. Humidity controller 200 includes a power controller (being all components shown in Figure 6 other than the heater plate 203 and heater plate temperature sensor 208) that can continuously control the level of power supplied to a heater plate 203 using a temperature signal 204 as the only transducer input. However, additional transducers may be used to achieve more refined control, such as using ambient temperature to modify a humidity profile as described above with reference to Figure 5. It will be appreciated that further transducer inputs may also be advantageously employed where more refined control is required. Other transducers may include ambient temperature sensors or sensors in the gas flow path, such as sensors that detect the flow rate, pressure, humidity or temperature of the humidified gases or other sensors.

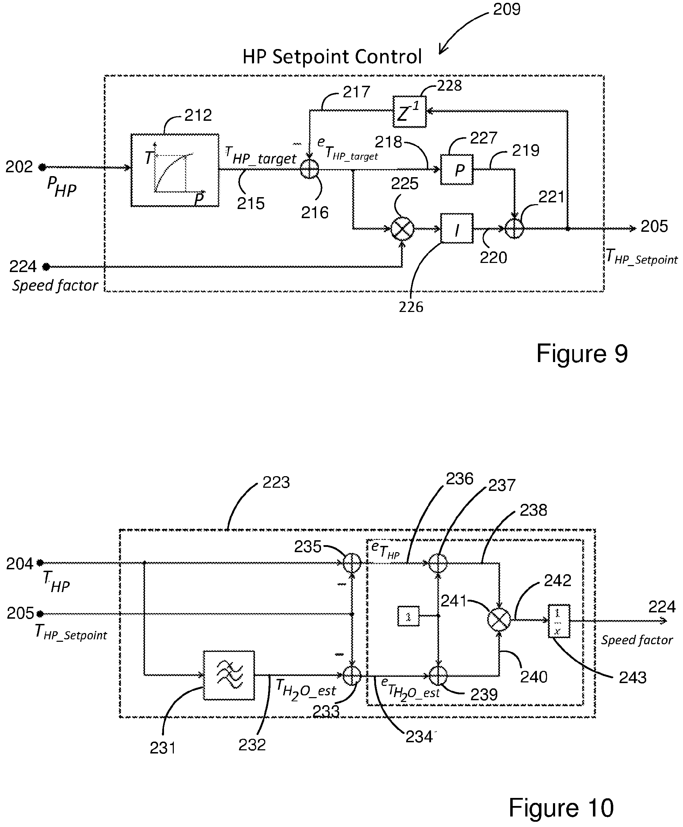

A user can select a humidity setting via button 134 (see Figure 1G) for a desired mode of therapy which is supplied to setpoint controller 209. Where multiple humidity profiles are provided a humidity profile associated with the selected humidity setting can be used to perform control as described below (i.e. the humidity profile, such as humidity profile 210 in Figure 7A, can be used by heater plate setpoint controller 209 as well as humidity profile 212 in Figure 9, although humidity profile 210 will be used to output a power level for a temperature input and humidity profile 212 will be used to output a temperature for a power input). Humidity profiles may comprise a continuous representation of values, such as a continuous curve or a relationship producing such values, such as a polynomial equation. Where a single humidity profile is used this may be modified according to the selected humidity setting to produce a humidity profile suitable to deliver the required humidity level. Alternatively, humidity profiles may consist of a set of discontinuous values, such as values stored in a look up table. Humidity controller 200 can continuously control the level of power supplied to a heater plate 203 based upon a heater plate temperature signal 204 and a heater plate temperature setpoint 205. In a first control loop a heater plate temperature controller 201 can control the level of a power signal 202 supplied to heater plate 203 based on heater plate temperature signal 204 and heater plate temperature setpoint 205. The heater plate temperature setpoint 205 may be initially set at a default value during a start-up phase of operation, 60 to 65 degrees Celsius for example. A heater plate temperature sensor 208 is configured to sense the temperature of the heater plate 203 (either directly or indirectly) and provide a temperature signal 204. Where temperature sensor 208 is a discrete sensor it may be located at or near the heater plate 203. For example, the temperature sensor 208 may be located underneath the heater plate 203. Where heating element resistance is used the temperature sensor can produce the temperature signal 204 by monitoring the resistance profile of a heating element of heater plate 203.

A heater plate temperature setpoint signal 205 is provided by a heater plate setpoint controller 209 in a second control loop. The difference or error between the temperature signal 204 and heater plate temperature setpoint 205 is determined at node 206 to produce an error signal 207 which is provided to heater plate temperature controller 201 to provide negative feedback control.

As shown in Figure 7A the heater plate temperature controller 201 can employ a PI controller to control the level of power supplied to the heater plate 203 based on proportional and integrated components of the error signal 207. The heater plate temperature controller 201 could alternatively employ any other suitable closed-loop controller such as one including proportional and/or integral and/or derivative components. For a PID controller including derivative components, the derivative component would typically be low in such a system. It will be appreciated that where proportional and/or derivative and/or integral components are employed that a PID controller may be employed with appropriate coefficients (i.e. a zero coefficient if a component is not employed at all or a small coefficient if a component has only a minor contribution). Whilst a controller may be referred to as a PI controller, for example, it will be appreciated that although the main components are proportional and integral that a minor derivative component may be employed.

The heater plate temperature controller 201 may optionally include a feedforward controller 210 which can modify the level of power supplied to the heater plate 203 based on an expected steady state power level required to be supplied to the heater plate 203 to achieve a desired target humidity level based on the current heater plate temperature setpoint value 205. Feedforward control enables a faster response during steady state operation by directly feeding the expected heater plate power for the given heater plate temperature setpoint to the output and relying less on the slower integral component. Feedforward controller 210 can use the humidity profile selected above defining heater plate temperature and heater plate power supply level combinations associated with a desired steady state humidity value (as per curve D shown in Figures 3 and 4). The desired steady state humidity values can be expressed as absolute humidity values.

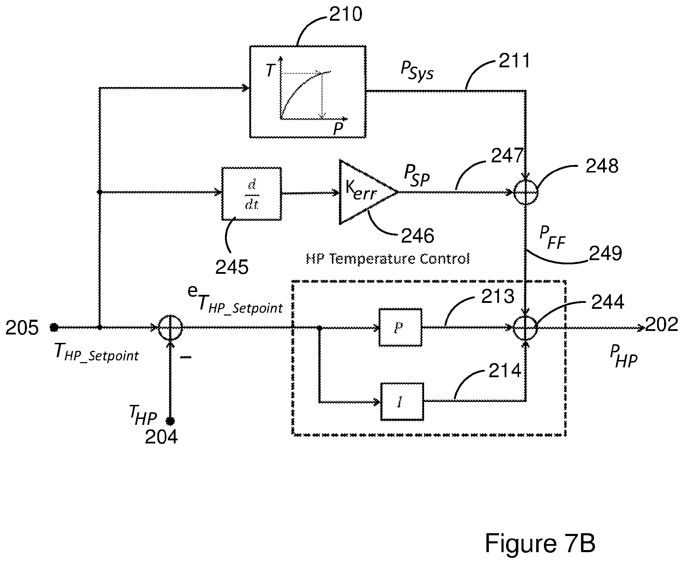

A modified form of feedforward control is shown in Figure 7B in which like elements have been given like numbers. In this modified controller an additional component 247 has been added to compensate for the additional power required to achieve a change in temperature of the water, which can be dependent upon the level of water in the humidifier chamber or based on a modelled water level, to reduce the burden of providing this power from other components. The additional component 247 is produced by differentiating the heater plate temperature setpoint value at 245 and scaling it at 246. This additional component 247 is added with component 211 at node 248 to produce a feedforward component 249 supplied to node 244. This modified form of feedforward control allows the heater plate temperature setpoint controller to reach a desired operating point more quickly.

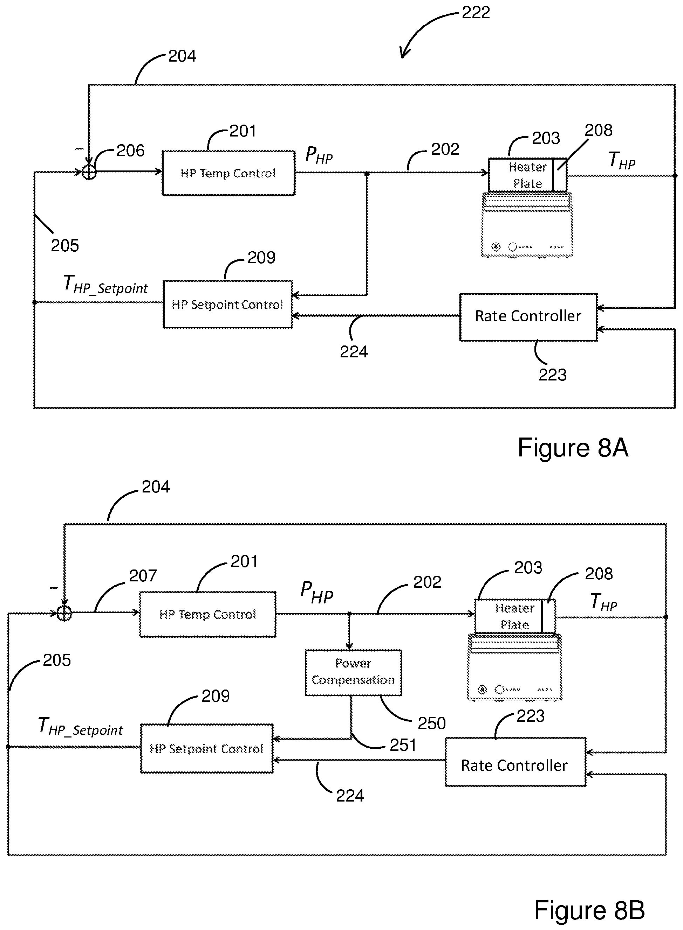

The feedforward component 211 from Figure 7A or 249 from Figure 7B may be combined with a feedback controller. In this example feedforward component 211 or 249 is combined with proportional component 213 of error signal 207 and an integral component 214 of error signal 207 at node 244 to produce power signal 202. Figure 6 and figure 8A show power signal 202 being inputted to HP setpoint control 209. In other embodiments, the HP setpoint control can receive a "steady state power signal" (i.e. not power signal 202) that corresponds to the combination of signal 211 and 214. The coefficients of the various signal components can be selected for a given application and environment.

Referring now to Figure 8A an example control diagram for a humidity controller 222 for a respiratory or surgical humidifier including the heater plate temperature setpoint controller of Figure 9 is shown. Humidity controller 222 is as per the controller of Figure 6 except that it includes a rate controller 223 which can variably control the rate of change of the heater plate temperature setpoint during a transient event, such as when water is added to the humidifier chamber.

With reference to Figure 9 an example heater plate temperature setpoint controller 209 for producing heater plate temperature setpoint 205 is shown. A target temperature generator 212 can produce a target temperature 215 based on the humidity profile selected above. The target heater plate temperature 215 can then be determined from the selected profile based on power signal 202, although for improved performance, such as reducing sensitivity to transients, steady state power may be used instead, for example; being the integral component 214 and the feedforward component 211. Buffer 228 provides an output 217 that is a delayed value of heater plate temperature setpoint 205. The difference between the target temperature 215 and output 217 corresponding to the prior heater plate temperature setpoint is determined at node 216 and output as error signal 218. A proportional block 227 outputs a scaled value (including a scale factor of 1) of error signal 218 as proportional component 219 and an integrator 226 outputs an integrated value of error signal 218 as an integral component 220. Proportional component 219 and integral component 220 are combined at node 221 to produce a new heater plate temperature setpoint 205. Purely integral control may also be used with proportional block 227 (and thus proportional component 219) omitted.

Figure 8B shows a modified form of the humidity controller 222 shown in Figure 8A in which like components have been given like numbering. In this case the heater plate power signal 202 (or "steady state power signal" as above) is modified by power compensator 250 to provide a compensated power signal 251 to heater plate setpoint controller 209. With decreasing volumes of water in the humidifier chamber there is decreased heat dissipation which, if not compensated since the system is based on a modelled water level, can result in a higher power level being inputted to the setpoint controller 209. This can in turn cause the setpoint controller 209 to lower the heater plate temperature setpoint 205, which can result in a lower humidity output. Power compensator 250 can modify the heater plate power signal being inputted to the setpoint controller 209 by compensating the power level to be equivalent to the power expected at the modelled water level. The water level used by power compensator 250 can be measured or inferred.

During certain events, for example at start-up or when water is added to a humidifier chamber, it may be desirable to avoid rapid change in the heater plate temperature setpoint to maintain stable operation and/or to more rapidly reach a desired operating point. In the above example the temperature of water in the humidifier chamber is not be measured directly and is based on heater plate temperature.

Transient events with large deviation from heater plate setpoint temperature (e.g. during start-up and when water is added) may cause the power output by the heater plate temperature controller to fluctuate rapidly. This can potentially lead to large changes in heater plate setpoint temperature, which can then cause even larger power fluctuations, thereby creating oscillations and potentially leading to instability.

Water exhibits a large specific heat capacity which causes a phase lag between actual water temperature and heater plate temperature. This is significant when there is a large volume of water present. For example, the water may be in the process of heating up and require a larger power input, but the heater plate temperature may already appear to be at the heater plate setpoint temperature.

It can thus be desirable to slow down the rate of change of the setpoint in such situations to avoid oscillations of the setpoint or instability and/or to more rapidly reach a desired operating point.

Referring again to Figures 8A and 8B, rate controller 223 produces a rate signal 224 supplied to heater plate setpoint controller 209. As seen in figure 9 the rate signal 224 is multiplied with the error signal 218 at node 225 to scale the error signal supplied to the integrator 226. The rate signal 224 can be viewed as a level of confidence that the heater plate power is at steady state. That is, a lower rate signal corresponds to a lower confidence and a higher rate signal corresponds to a higher confidence. This reduces the amount of change of the heater plate temperature setpoint 205 due to the integral component 220 in dependence upon rate signal 224.

Referring to Figure 10 an example rate controller 223 for use in the power controller shown in Figure 8A or 8B is shown. A heater plate temperature signal 204 and a heater plate temperature setpoint signal 205 are supplied as inputs to rate controller 223.

In a lower branch the heater plate temperature signal 204 is filtered by low pass filter 231 to provide an estimated water temperature signal 232 (T_{H2O_est}). At node 233 the heater plate temperature setpoint value 205 is subtracted from the estimated water temperature signal 232 to produce an estimated water temperature error signal 234 (e_{TH2O_est}). This branch may affect the rate signal 224 so as to reduce the rate of change of the heater plate temperature setpoint so as to reflect the effect of the lag in water temperature behind heater plate temperature.

In an upper branch at node 235 the heater plate temperature setpoint value 205 is subtracted from the heater plate temperature signal 204 to produce a heater plate temperature error signal 236 (e_{THP}). This branch may affect the rate signal 224 so as to reduce the rate of change of the heater plate temperature setpoint so as to exclude or at least partially reduce the effects of periods where the system has not reached steady-state operation at the given heater plate setpoint temperature.

At nodes 237 and 239 the value 1 is added to both the estimated water temperature error signal 234 (e_{TH2O_est}) and the heater plate temperature error signal 236 (e_{THP}) so they do not cancel out each other to give a divide-by-zero condition at node 243. These signals 238 and 240 are multiplied by multiplier 241 to produce signal 242. The reciprocal of signal 242 is output by node 243 as rate signal 224 which is represented by the equation below: $\frac{1}{\left({e}_{{T}_{HP}}+1\right) \left({e}_{{TH}_{2} O _ est}+1\right)}$

It will be seen that the rate signal 224 decreases as either error signal e_{THP} or e_{TH2O_est} increases. That is, the rate signal 224 is negatively related to both the error signal e_{THP} and e_{TH₂O_est}.

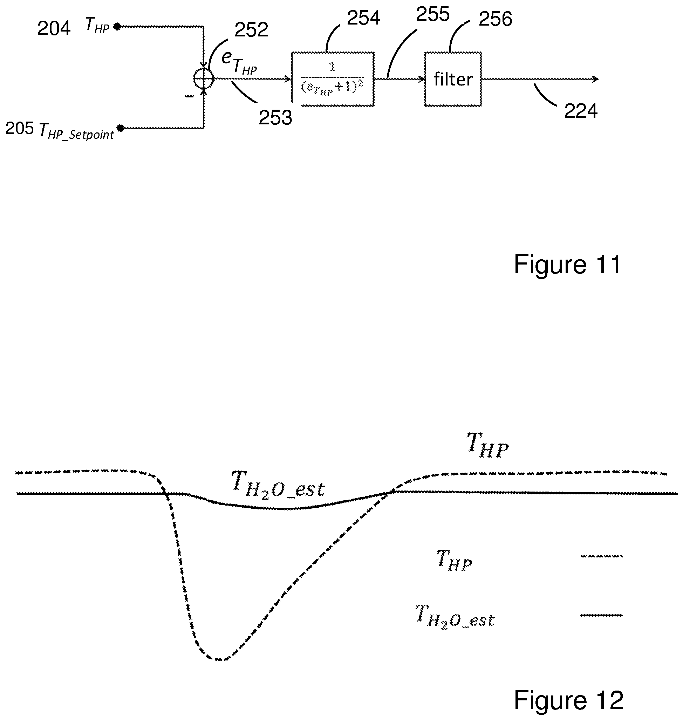

An alternative form of rate controller is shown in Figure 11 which may provide a rate signal 224 to the power controller shown in Figure 8A or 8B. In this example the heater plate temperature setpoint value 205 is subtracted from the heater plate temperature signal 204 at node 252 to produce an error signal 253. The error signal is operated upon at block 254 by a polynomial function to produce an output signal 255, which is filtered by filter 256 to produce a rate signal 224. The filter 256 can be an asymmetric filter. The polynomial function employed at block 254 could be any of a number of suitable quadratic, cubic or other functions. Any other suitable function may be employed, such as an exponential function. An example function for blocks 254 and 256 is: $rate signal = filter \left(\frac{1}{{\left({e}_{{T}_{HP}}+1\right)}^{2}}\right)$ where *e_{T_{HP}}* is the error signal 253.

Using this equation the rate signal 253 reduces quadratically with the magnitude of error. This equation results in similar behaviour to the example shown in Figure 10 and does not require an estimated water temperature value to be produced. Where this example rate controller is used with the heater plate setpoint controller 209 of Figure 9 without proportional control (i.e. omitting block 227) the rate signal may be viewed as a confidence measure where the integral component (block 226) performs "normal" control when the rate signal indicates a high confidence level (i.e. the rate signal is close to 1) and integral control is 'slowed down' with lower confidence levels (i.e. the rate signal is low).

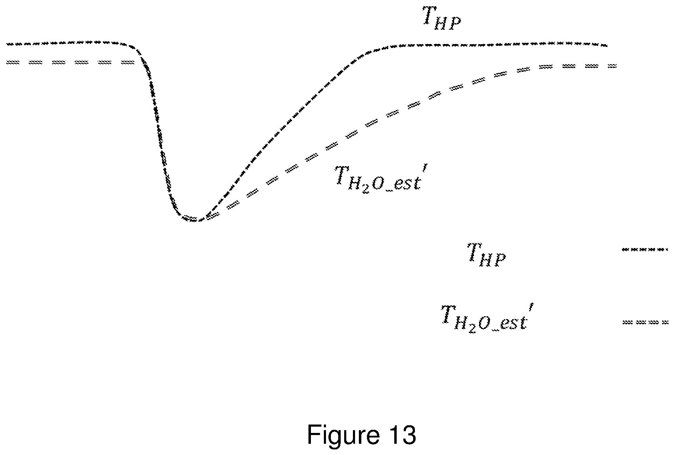

Ideally T_{H2O_est} would equal T_{HP}. In some situations, such as when cold water is added to the humidifier chamber, T_{H2O_est} may exhibit too much lag; that is, it may fail to estimate the sudden drop in water temperature, as illustrated in Figure 12. In this case the heater plate temperature T_{HP} is seen to sharply decrease but the low pass filtered estimated water temperature T_{H2O_est} maintains a relatively constant value over the transient period and so does not drive a rapid response to the sharp decrease in heater plate temperature. In this situation a modified control strategy can be employed as illustrated in Figure 13. Under this modified control strategy the modified low pass filtered estimated water temperature T_{H2O_est}' behaves as an asymmetric filter, as follows:
Phase 1: When the output of the low-pass filter 231 is greater than the heater plate temperature T_{HP} the output of the low pass filter is set to be equal to the heater plate temperature (i.e. the period from when the heater plate curve T_{HP} passes below the filtered estimated water temperature T_{H2O_est} to the point of inflection at the bottom of the heater plate temperature curve T_{HP}); and
Phase 2: When the output of the low-pass filter 231 is less than the heater plate temperature T_{HP} the output of the low pass filter is used (starting from the heater plate temperature value at inflection).

The above examples and controllers may be implemented as microprocessor-based systems or circuits using discrete circuit components. One or more humidity profiles may be stored in non-volatile memory in the form of a relationship producing a continuous curve, such as defined by a polynomial equation. Alternatively, the humidity profile may by represented by a piecewise function. A relationship for modifying the one or more humidity profiles based on ambient temperature may also be stored. Alternatively, discontinuous values, such as a series of pairs of heat transfer body temperature and heater power supply level combinations delivering a desired steady state humidity value may be stored in suitable memory, such as in a look up table.

Methods and processes described herein may be embodied in, and partially or fully automated via, software code modules executed by one or more general and/or special purpose computers. The word "module" refers to logic embodied in hardware and/or firmware, or to a collection of software instructions, possibly having entry and exit points, written in a programming language, such as, for example, C or C++. A software module may be compiled and linked into an executable program, installed in a dynamically linked library, or may be written in an interpreted programming language such as, for example, BASIC, Perl, or Python. It will be appreciated that software modules may be callable from other modules or from themselves, and/or may be invoked in response to detected events or interrupts. Software instructions may be embedded in non-volatile memory, such as an erasable programmable read-only memory (EPROM). It will be further appreciated that hardware modules may comprise connected logic units, such as gates, flip-flops and/or application specific integrated circuits, and/or may comprise programmable units, such as programmable gate arrays and/or processors. The modules described herein can be implemented as software modules, but also may be represented in hardware and/or firmware. Moreover, although in some embodiments a module may be separately compiled, in other embodiments a module may represent a subset of instructions of a separately compiled program and may not have an interface available to other logical program units.

In certain embodiments, code modules may be implemented and/or stored in any type of computer-readable medium or other computer storage device. In some systems, data (and/or metadata) input to the system, data generated by the system, and/or data used by the system can be stored in any type of computer data repository, such as a relational database and/or flat file system. Any of the systems, methods, and processes described herein may include an interface configured to permit interaction with users, operators, other systems, components, programs, and so forth.

It should be emphasized that many variations and modifications may be made to the embodiments described herein, the elements of which are to be understood as being among other acceptable examples. All such modifications and variations are intended to be included herein within the scope of this disclosure and protected by the following claims. Further, nothing in the foregoing disclosure is intended to imply that any particular component, characteristic or process step is necessary or essential.

It will thus be appreciated that there is disclosed:
1. A respiratory or surgical humidifier for delivering gases at a desired level of humidity and/or a desired temperature comprising:
   a. a housing configured to receive a humidifier chamber;
   b. a heating assembly located at least partially within the housing, the heating assembly including:
      i. a heat transfer body configured to transfer heat to the humidifier chamber when the humidifier chamber is received by the housing;
      ii. a heat transfer body temperature sensor configured to sense the temperature of the heat transfer body; and
      iii. a heater configured to heat the heat transfer body; and
   c. a power controller configured to control a level of power supplied to the heater in dependence upon a temperature signal received from the heat transfer body temperature sensor and one or more humidity profiles defining heat transfer body temperature and heater power supply level combinations associated with a desired humidity value.
2. A respiratory or surgical humidifier as clause 1 wherein the heat transfer body temperature and heater power supply level combinations of each humidity profile form a curve associated with a constant humidity value over a desired operating range of the humidifier.
3. A respiratory or surgical humidifier as clause 1 or clause 2 wherein each humidity profile describes a relationship between heat transfer body temperatures and heater power supply levels delivering a desired constant humidity value over a desired operating range of the humidifier.
4. A respiratory or surgical humidifier clause 1 wherein the heat transfer body temperature and heater power supply level combinations of each humidity profile consist of a plurality of discrete heat transfer body temperature and heater power supply level combinations delivering a desired constant humidity value over a desired operating range of the humidifier.
5. A respiratory or surgical humidifier as any one of clauses 1 to 4 wherein each humidity profile may be selected from one of a number of profiles for different constant humidity values.
6. A respiratory or surgical humidifier as any one of the preceding clauses wherein the power controller controls the level of power supplied to the heater in dependence only upon temperature signals received from the heat transfer body temperature sensor.
7. A respiratory or surgical humidifier as any one of clauses 1 to 5 including an ambient temperature sensor.
8. A respiratory or surgical humidifier clause 6 wherein the ambient temperature sensor is selected from: an infrared detector, a negative temperature coefficient thermistor and a positive temperature coefficient thermistor.
9. A respiratory or surgical humidifier as clause 6 or clause 7 wherein each humidity profile is modified based on ambient temperature.
10. A respiratory or surgical humidifier as clause8 wherein each humidity profile is scaled based on ambient temperature.
11. A respiratory or surgical humidifier as any one of the preceding clauses wherein the desired humidity value is a substantially constant steady state humidity value.
12. A respiratory or surgical humidifier as any one of the preceding clauses including non-volatile memory storing one or more humidity profiles.
13. A respiratory or surgical humidifier as any one of the preceding clauses wherein the power controller continuously controls the level of power supplied to the heater.
14. A respiratory or surgical humidifier as any one of the preceding clauses wherein the power controller includes a heater control circuit which varies the level of power supplied to the heater at least in part in dependence upon the difference between the temperature signal and a heater plate temperature setpoint value.
15. A respiratory or surgical humidifier as clause 14 wherein the heater control circuit varies the level of power supplied to the heater at least in part in dependence upon proportional and/or integral and/or derivative components of the difference between the temperature signal and a heater plate temperature setpoint value.
16. A respiratory or surgical humidifier as clause 15 wherein the heater control circuit includes a feed forward circuit which modifies the level of power supplied to the heater based on an expected steady state power level for the heater plate temperature setpoint value.
17. A respiratory or surgical humidifier as clause 16 wherein the expected steady state power level is determined by finding the power level associated with the heater plate temperature setpoint value in the humidity profile.
18. A respiratory or surgical humidifier as clause 17 wherein the power level from the humidity profile is modified by a derivative value of the heater plate temperature setpoint value.
19. A respiratory or surgical humidifier as clause 18 wherein a derivative value of the heater plate temperature setpoint value is added to the power level from the humidity profile.
20. A respiratory or surgical humidifier as any one of clauses 14 to 19 wherein the power controller includes a heater plate temperature setpoint controller that produces a heater plate temperature setpoint value based at least in part upon the heater power supply level or one or more components thereof and a rate signal based on temperature information.
21. A respiratory or surgical humidifier as clause 20 wherein the heater plate temperature setpoint controller develops a target temperature based on a target temperature associated with the heater power supply level in the humidity profile and modifies the heater plate temperature setpoint based at least in part on the target temperature and the rate signal.
22. A respiratory or surgical humidifier as clause 21 wherein the heater plate temperature setpoint controller determines the difference between a prior heater plate temperature setpoint and a target heater plate temperature setpoint and integrates this difference to produce a new heater plate temperature setpoint value.
23. A respiratory or surgical humidifier as clause 21 wherein the heater plate temperature setpoint controller determines the difference between a prior heater plate temperature setpoint and a target heater plate temperature setpoint and combines proportional and integrated components of this difference to produce a new heater plate temperature setpoint value.
24. A respiratory or surgical humidifier as any one of clauses 20 to 23 wherein the heater power supply level or one or more components thereof supplied to the heater plate temperature setpoint controller are adjusted in dependence upon humidifier chamber fluid level.
25. A respiratory or surgical humidifier as clause 24 wherein the heater power supply level supplied to the heater plate temperature setpoint controller is increased with lowering humidifier chamber fluid level.
26. A respiratory or surgical humidifier as any one of clause 22 to 25 wherein the rate signal modifies the difference between a prior heater plate temperature setpoint and a target heater plate temperature setpoint prior to integration.
27. A respiratory or surgical humidifier as any one of clause 20 to 26 wherein the rate signal is based at least in part on the temperature signal and the heater plate temperature setpoint value.
28. A respiratory or surgical humidifier as clause 27 wherein the rate signal reduces the rate of change of the integral component in dependence upon the absolute difference between the temperature signal and heater plate temperature setpoint value.
29. A respiratory or surgical humidifier as any one of clauses 20 to 28 wherein the heater plate temperature setpoint is a predetermined value at start-up.
30. A respiratory or surgical humidifier as any one of the preceding clauses wherein the heater is a heating element.
31. A respiratory or surgical humidifier as clause 30 wherein the heating element is a resistive heating element.
32. A respiratory or surgical humidifier as clause 31 wherein the heating element is formed of nichrome wire.
33. A respiratory or surgical humidifier as clauses 31 or 32 wherein the heating element is wound and provided within or in thermal contact with the heat transfer body.
34. A respiratory or surgical humidifier as any one of clauses 30 to 33 wherein the heat transfer body temperature sensor produces the temperature signal based on a resistance profile of the heating element.
35. A respiratory or surgical humidifier as any one of clauses 1 to 33 wherein the heat transfer body temperature sensor is a negative temperature coefficient thermistor.
36. A respiratory or surgical humidifier as n any one of clauses 1 to 33 wherein the heat transfer body temperature sensor is a positive temperature coefficient thermistor.
37. A respiratory or surgical humidifier as any one of clauses 1 to 33 wherein the heat transfer body temperature sensor is a thermocouple.
38. A respiratory or surgical humidifier as any one of clauses 1 to 33 wherein the heat transfer body temperature sensor is an infrared sensor.
39. A respiratory or surgical humidifier as any one of the preceding clauses including two heat transfer body temperature sensors.
40. A respiratory or surgical humidifier as any one of the preceding clauses wherein the power controller includes one or more microprocessor.
41. A respiratory or surgical humidifier as any one of the preceding clauses wherein the heat transfer body is a heater plate.
42. A respiratory or surgical humidifier as any one of the preceding clauses including a humidifier chamber having an inlet for receiving gases and an outlet for supplying humidified gases.
43. A respiratory or surgical humidifier for delivering gases at a desired level of humidity and/or a desired temperature comprising:
   a. a housing configured to receive a humidifier chamber;
   b. a heating assembly located at least partially within the housing, the heating assembly including:
      i. a heat transfer body configured to transfer heat to the humidifier chamber when the humidifier chamber is received by the housing;
      ii. a heat transfer body temperature sensor configured to sense the temperature of the heat transfer body and provide a temperature signal; and
      iii. a heater configured to heat the heat transfer body; and
   c. a power controller configured to:
      i. determine a heater plate temperature setpoint required for the humidifier to deliver gases at a desired level of humidity based at least in part on a level of power supplied to the heater and the temperature signal, wherein a rate of change of the heater plate temperature setpoint is variably controlled in dependence at least in part upon the difference between the heater plate temperature setpoint and the temperature signal; and
      ii. control the level of power supplied to the heater based on the heater plate temperature setpoint and the temperature signal.
44. A respiratory or surgical humidifier as clause 43 wherein the rate of change of the heater plate temperature setpoint is controlled in dependence upon a rate signal which is based on the current heater plate temperature setpoint and both the temperature signal and an estimated temperature of a fluid within a humidifier chamber when in use.
45. A respiratory or surgical humidifier as clause 44 wherein the estimated temperature is obtained by low pass filtering the temperature signal.
46. A respiratory or surgical humidifier as clause 44 wherein the estimated temperature is obtained by using the heater plate temperature signal when the low pass filtered signal is greater than the heater plate temperature and using the low pass filtered signal when the low pass filtered signal is less than the heater plate temperature.
47. A respiratory or surgical humidifier as any one of clauses43 to 46 wherein both the temperature signal and the estimated temperature are compared with the heater plate temperature setpoint to produce error signals which are combined to produce a rate signal controlling the rate of change of the heater plate temperature setpoint.
48. A respiratory or surgical humidifier as clause 43 wherein the rate of change of the heater plate temperature setpoint is controlled in dependence upon the current heater plate temperature setpoint and the temperature signal.
49. A respiratory or surgical humidifier as clause 48 wherein a polynomial function is applied to the difference between the current heater plate temperature setpoint and the temperature signal and inverted to produce the rate signal.
50. A respiratory or surgical humidifier as clause 48 wherein an exponential function is applied to the difference between the current heater plate temperature setpoint and the temperature signal and inverted to produce the rate signal.
51. A respiratory or surgical humidifier as any one of clause 48 to 50 wherein a constant is added to the difference between the current heater plate temperature setpoint and the temperature signal and squared and then inverted to produce the rate signal.
52. A respiratory or surgical humidifier as any one of clause 49 to 51 wherein the rate signal is filtered.
53. A respiratory or surgical humidifier as clause 52 wherein the rate signal is filtered by an asymmetric filter.
54. A respiratory or surgical humidifier as any one of clauses 43 to 53 wherein the power controller includes a heater plate temperature setpoint controller that produces a heater plate temperature setpoint value based at least in part upon the heater power supply level and the rate signal.
55. A respiratory or surgical humidifier as clause 54 wherein the heater plate temperature setpoint controller develops a target temperature based on a target temperature associated with a heater power level of the humidity profile and modifies the target temperature based at least in part on the rate signal to produce a heater plate temperature setpoint.
56. A respiratory or surgical humidifier as clause 55 wherein the heater plate temperature setpoint controller determines the difference between a prior heater plate temperature setpoint and a target heater plate temperature setpoint and integrates this difference to produce a new heater plate temperature setpoint value.
57. A respiratory or surgical humidifier as clause 55 wherein the heater plate temperature setpoint controller determines the difference between a prior heater plate temperature setpoint and a target heater plate temperature setpoint to produce a difference signal and combines proportional and integral components of the difference signal to produce a new heater plate temperature setpoint.
58. A respiratory or surgical humidifier as clause 56 or 57 wherein the rate signal modifies an integral input used to produce the integrated component.
59. A respiratory or surgical humidifier as clause 58 wherein the rate signal reduces the integral input with increasing difference between the temperature signal and heater plate temperature setpoint value.
60. A respiratory or surgical humidifier as clause 58 wherein the rate signal reduces the integral input with increasing difference between the heater plate temperature setpoint value and both the temperature signal and estimated temperature.
61. A respiratory or surgical humidifier as any one of clauses 43 to 60 wherein the power controller is configured to control a level of power supplied to the heater in dependence upon temperature signals received from the heat transfer body temperature sensor and one or more humidity profiles defining heat transfer body temperature and heater power supply level combinations associated with a desired humidity value.
62. A respiratory or surgical humidifier as clause 61 wherein the heat transfer body temperature and heater power supply level combinations form a curve associated with a constant humidity value over a desired operating range of the humidifier.
63. A respiratory or surgical humidifier as clause 61 or clause 62 wherein the humidity profile may be selected from one of a number of profiles for different constant humidity values.
64. A respiratory or surgical humidifier as any one of clauses 60 to 63 including an ambient temperature sensor.
65. A respiratory or surgical humidifier as clause 64 wherein the ambient temperature sensor is an infrared detector, a negative temperature coefficient thermistor or a positive temperature coefficient thermistor.
66. A respiratory or surgical humidifier as clause 64 or clause 65 wherein the one or more humidity profiles are modified based on ambient temperature.
67. A respiratory or surgical humidifier as clause 66 wherein the one or more humidity profiles are scaled based on ambient temperature.
68. A respiratory or surgical humidifier as any one of clauses 60 to 67 wherein the desired humidity value is a substantially constant steady state humidity value.
69. A respiratory or surgical humidifier as any one of clauses 60 to 68 including non-volatile memory storing the one or more humidity profiles.
70. A respiratory or surgical humidifier as any one of clauses 60 to 69 wherein the power controller continuously controls the level of power supplied to the heater.
71. A respiratory or surgical humidifier as any one of clauses 60 to 70 wherein the power controller controls the level of power supplied to the heater in dependence upon only one monitored variable, being a temperature signal received from the heat transfer body temperature sensor.
72. A respiratory or surgical humidifier as any one of clause 43 to 71 wherein the heater is a heating element.
73. A respiratory or surgical humidifier as clause 72 wherein the heating element is a resistive heating element.
74. A respiratory or surgical humidifier as clause 73 wherein the heating element is formed of nichrome wire.
75. A respiratory or surgical humidifier as clause 73 or 74 wherein the heating element is wound and provided within or in thermal contact with the heat transfer body.
76. A respiratory or surgical humidifier as any one of clause 72 to 75 wherein the heat transfer body temperature sensor produces the temperature signal based on a resistance profile of the heating element.
77. A respiratory or surgical humidifier as any one of clauses 43 to 71 wherein the heat transfer body temperature sensor is a negative temperature coefficient thermistor.
78. A respiratory or surgical humidifier as any one of clauses 43 to 71 wherein the heat transfer body temperature sensor is a positive temperature coefficient thermistor.
79. A respiratory or surgical humidifier as any one of clauses 43 to 71 wherein the heat transfer body temperature sensor is a thermocouple.
80. A respiratory or surgical humidifier as clause any one of clauses 43 to 71 wherein the heat transfer body temperature sensor is an infrared sensor.
81. A respiratory or surgical humidifier as any one of clauses 43 to 80 wherein the heat transfer body temperature sensor comprises two temperature sensors.
82. A respiratory or surgical humidifier as any one of clauses 43 to 81 wherein the power controller includes one or more microprocessors.
83. A respiratory or surgical humidifier as any one of clauses 43 to 82 wherein the heat transfer body is a heater plate.
84. A respiratory or surgical humidifier as any one of clauses43 to 83 including a humidifier chamber having an inlet for receiving gases and an outlet for supplying humidified gases.
85. A respiratory or surgical humidifier for delivering gases at a desired level of humidity and/or a desired temperature comprising:
   a. a housing configured to receive a humidifier chamber;
   b. a heating assembly located at least partially within the housing, the heating assembly including:
      i. a heat transfer body configured to transfer heat to the humidifier chamber when the humidifier chamber is received by the housing;
      ii. a heat transfer body temperature sensor configured to sense the temperature of the heat transfer body and provide a temperature signal; and
      iii. a heater configured to heat the heat transfer body; and
   c. a power controller configured to:
      i. determine a heater plate temperature setpoint required for the humidifier to achieve a desired operating point based at least in part on the temperature signal and a level of power supplied to the heater wherein a rate of change of the heater plate temperature setpoint is variably controlled in dependence at least in part upon the difference between the heater plate temperature setpoint and the temperature signal; and
      ii. control the level of power supplied to the heater in dependence upon the temperature signal and one or more humidity profiles defining heat transfer body temperature and heater power supply level combinations associated with a desired humidity value.
86. A method of controlling a respiratory or surgical humidifier so as to maintain gases at a desired humidity level and/or a desired temperature by controlling the temperature of a heat source heating a liquid in a chamber based at least in part on a heat source temperature setpoint determined in dependence upon:
   a. power supplied to the heat source;
   b. heat source temperature; and
   c. one or more humidity profiles defining heat source temperature and heat source power supply level combinations associated with a respective desired humidity value,
   so as to drive an operating point of the humidifier towards a combination of values of the or a selected humidity profile.
87. A method as clause 86 wherein the or a selected humidity profile defines a curve and the level of power is controlled to drive an operating point towards the curve.
88. A respiratory or surgical humidifier for delivering gases at a desired level of humidity and/or a desired temperature comprising:
   a. a housing configured to receive a humidifier chamber; and
   b. a heater plate assembly located at least partially within the housing, the heater plate assembly including:
      i. a heater plate configured to contact a base of the humidifier chamber when the humidifier chamber is received by the housing;
      ii. a heater plate temperature sensor located at or near the heater plate; and
      iii. a heating element configured to heat the heater plate; and
   c. a power controller configured to control a level of power supplied to the heating element in dependence upon the temperature signal and one or more humidity profile defining heat transfer body temperature and heater power supply level combinations associated with a respective desired humidity value.
89. A respiratory or surgical humidifier for delivering gases at a desired level of humidity and/or a desired temperature comprising:
   a. a housing configured to receive a humidifier chamber; and
   b. a heater plate assembly located at least partially within the housing, the heater plate assembly including:
      i. a heater plate configured to contact a base of the humidifier chamber when the humidifier chamber is received by the housing;
      ii. a heater plate temperature sensor located at or near the heater plate; and
      iii. a heating element configured to heat the heater plate; and
   c. a power controller including:
      i. a temperature monitoring circuit configured to determine heater plate temperature based on signals received from the heater plate temperature sensor;
      ii. memory storing one or more humidity profiles defining heating plate temperature and heating element power supply level combinations associated with a respective desired humidity value; and
      iii. a power level controller configured to control the level of power supplied to the heating element based upon power level and heater plate temperature values so as to drive an operating point of the humidifier towards the or a selected humidity profile to achieve the desired humidity value.
90. A respiratory or surgical humidifier for delivering gases at a desired level of humidity and/or a desired temperature comprising:
   a. a housing configured to receive a humidifier chamber; and
   b. a heater plate assembly located at least partially within the housing, the heater plate assembly including:
      i. a heater plate configured to contact a base of the humidifier chamber when the humidifier chamber is received by the housing;
      ii. a heater plate temperature sensor configured to sense the temperature of the heater plate and provide a temperature signal; and
      iii. a heating element configured to heat the heater plate; and
   c. a power controller configured to:
      i. determine a heater plate temperature setpoint required for the humidifier to achieve a desired operating point based on the temperature signals and a power level applied to the heating element by:
         a. determining a target temperature of the heater plate based on the power level;
         b. determining an error signal based on a difference between the target temperature and the current heater plate temperature setpoint;
         c. developing a new heater plate temperature setpoint based on proportional and/or integral values of the error signal, wherein the error signal is modified in dependence upon the heater plate temperature setpoint and the temperature signal before integration; and
      ii. controlling the level of power supplied to the heating plate based on the heater plate temperature setpoint and temperature signal.
91. A respiratory or surgical humidifier for delivering gases at a desired level of humidity and/or a desired temperature comprising:
   a. a housing configured to receive a humidifier chamber; and
   b. a heater plate assembly located at least partially within the housing, the heater plate assembly including:
      i. a heater plate configured to contact a base of the humidifier chamber when the humidifier chamber is received by the housing;
      ii. a heater plate temperature sensor configured to sense the temperature of the heater plate and provide a temperature signal; and
      iii. a heating element configured to heat the heater plate; and
   c. a power controller configured to:
      i. determine a heater plate temperature setpoint required for the humidifier to achieve a desired operating point based on temperature signals and a power level applied to the heating element by:
         a. determining a target temperature of the heater plate based on a defined relationship with the power signal;
         b. developing an error signal based on a difference between the target temperature and the temperature signal; and
         c. developing a heater plate temperature setpoint based on proportional and/or integrated values of the error signal, wherein the error signal is modified in dependence upon the difference between the heater plate temperature setpoint and the temperature signal and/or a filtered temperature signal before integration; and
      ii. controlling the level of power supplied to the heating element based on the heater plate temperature setpoint and temperature signal.
92. A respiratory or surgical humidifier for delivering gases at a desired level of humidity and/or a desired temperature comprising:
   a. a heater configured to transfer heat to a humidifier chamber;
   b. a temperature sensor configured to sense the temperature of the heater; and
   c. a power controller configured to continuously control a level of power supplied to the heater in dependence upon only one monitored variable, being signals received from the temperature sensor.
93. A respiratory or surgical humidifier as clause 92 wherein the power controller stores one or more humidity profiles defining heater temperature and heater power supply level combinations associated with a desired humidity value and drives operation of the humidifier towards values stored in the humidity profile.
94. A respiratory or surgical humidifier or method as any one of the preceding clauses wherein the desired level of humidity is a desired level of absolute humidity.

## Claims

1. A respiratory or surgical humidifier (1; 100) for delivering gases at a desired level of humidity and/or a desired temperature comprising:
a. a housing configured to receive a humidifier chamber;
b. a heating assembly located at least partially within the housing, the heating assembly including:
i. a heater plate (120; 203) configured to transfer heat to the humidifier chamber when the humidifier chamber is received by the housing;
ii. a heater plate temperature sensor (208) configured to sense the temperature of the heater plate (102; 203); and
iii. a heater configured to heat the heater plate (102; 203); and
c. a power controller configured to control a level of power supplied to the heater in dependence upon a temperature signal (204) received from the heater plate temperature sensor (208) and one or more humidity profiles (212) defining heater plate temperature and heater power supply level combinations associated with a desired humidity value;
wherein the power controller includes a heater plate temperature setpoint controller (209);
and wherein the heater plate temperature setpoint controller (209):
produces a heater plate temperature setpoint value based at least in part on the heater power supply level or one or more components thereof and a rate signal (224); and
develops a target temperature (215) based on a temperature associated with the heater power supply level in the humidity profile (212) and modifies the heater plate temperature setpoint based at least in part on the target temperature (215) and the rate signal (224);
wherein the rate signal (224) is based at least in part on the temperature signal and the heater plate temperature setpoint value.

2. A respiratory or surgical humidifier as claimed in claim 1 wherein a polynomial function is applied to a difference between the current heater plate temperature setpoint and the temperature signal and inverted to produce the rate signal.

3. A respiratory or surgical humidifier as claimed in claim 1 or 2 wherein an exponential function is applied to the difference between the current heater plate temperature setpoint and the temperature signal to produce the rate signal.

4. A respiratory or surgical humidifier as claimed in any one of claims 1 to 3 wherein the rate signal is filtered by an asymmetric filter.

5. A respiratory or surgical humidifier as claimed in any one of claims 1 to 4 wherein the heater plate temperature setpoint controller determines the difference between a prior heater plate temperature setpoint and a target heater plate temperature setpoint and integrates this difference to produce a new heater plate temperature setpoint value.

6. A respiratory or surgical humidifier as claimed in any one of claims 1 to 4 wherein the heater plate temperature setpoint controller determines the difference between a prior heater plate temperature setpoint and a target heater plate temperature setpoint and combines proportional and integrated components of this difference to produce a new heater plate temperature setpoint value.

7. A respiratory or surgical humidifier as claimed in claim 5 or 6 wherein the rate signal modifies the difference between a prior heater plate temperature setpoint and a target heater plate temperature setpoint prior to integration.

8. A respiratory or surgical humidifier as claimed in any one of claims 1 to 7 wherein each humidity profile may be selected from one of a number of profiles for different constant humidity values, the respiratory or surgical humidifier including non-volatile memory storing one or more humidity profiles.

9. A respiratory or surgical humidifier as claimed in any one of claims 1 to 8 including an ambient temperature sensor, wherein each humidity profile is modified based on ambient temperature.

10. A respiratory or surgical humidifier as claimed in any one of claims 1 to 8 wherein the power controller controls the level of power supplied to the heater in dependence only upon temperature signals received from the heater plate temperature sensor.

11. A respiratory or surgical humidifier as claimed in any one of claims 1 to 10 wherein the power controller is configured to control the level of power supplied to the heater plate without requiring use of a flow rate of the gases.

12. A respiratory or surgical humidifier (1; 100) as claimed in any one of claims 1 to 10 wherein the power controller is configured to control the level of power supplied to the heater plate (120; 203) without additional sensors in: a) the humidifier chamber; b) a gases outlet of the humidifier chamber; and/or c) a breathing circuit connected to the gases outlet of the humidifier chamber.

13. A respiratory or surgical humidifier as claimed in any one of claims 1 to 12 wherein the power controller is configured to determine the target temperature without needing to determine a flow rate of the gases.

14. A respiratory or surgical humidifier as claimed in any one of claims 1 to 13 wherein the humidity profile outputs the temperature based on the level of power supplied to the heater.

15. A respiratory or surgical humidifier as claimed in any one of claims 1 to 14, wherein the rate signal controls the rate of change of the heater plate temperature setpoint.
